# FASCICULE DE BREVET EUROPEEN

(11) **EP 2 770 852 B1**
(45) Date de publication et mention de la délivrance du brevet: **28.12.2016**
(21) Numéro de dépôt: 12790443.1
(22) Date de dépôt: 25.10.2012
(51) Int. Cl.: A61K 38/01, A23L 33/17

(54) **PRODUIT DIETETIQUE DESTINE A LA DIMINUTION DE LA GRAISSE VISCERALE EN PHASE PREOPERATOIRE BARIATRIQUE**
DIÄTETISCHES MITTEL ZUR VERMINDERUNG DER VISZERALEN FETTMENGE IN EINER PREOPERATIVEN PHASE
DIETETIC PRODUCT FOR REDUCING VISCERAL FAT IN BARIATRIC PREOPERATIVE PHASE

(30) Priorité: 25.10.2011 FR 1159664
(43) Date de publication de la demande: 03.09.2014
(73) Titulaire: International Nutrition Research Company, 1130 Luxembourg (LU)
(72) Inventeur: VINCENT, Claude, F-33000 Bordeaux (FR)
(74) Mandataire: Aquinov
(86) Numéro de dépôt international: PCT/EP2012/071114
(87) Numéro de publication internationale: WO 2013/060759

(56) Documents cités:
- EP-A1- 2 149 369
- WO-A1-2004/014152
- WO-A1-2010/047581
- WO-A1-2010/114627
- WO-A2-2007/022312
- WO-A2-2009/115331
- WO-A2-2010/043415
- FR-A1- 2 902 607
- US-A1- 2009 181 145
- LOURENCO DA COSTA ET AL: "Effect of heat and enzymatic treatment on the antihypertensive activity of whey protein hydrolysates", INTERNATIONAL DAIRY JOURNAL, ELSEVIER APPLIED SCIENCE, BARKING, GB, vol. 17, no. 6, 20 février 2007 (2007-02-20), pages 632-640, XP005895471, ISSN: 0958-6946, DOI: 10.1016/J.IDAIRYJ.2006.09.003

## Description

La présente invention concerne un produit diététique et son utilisation en particulier pour faire diminuer la graisse viscérale chez des patients obèses, en phase préopératoire d'une intervention chirurgicale bariatrique.

Le nombre d'individus obèses, plus particulièrement dans les pays développés, ne cesse de s'accroître.

Les risques entraînés par l'obésité sont nombreux. En particulier, on sait que l'obésité augmente le risque cardiométabolique, qui désigne la présence chez un individu de plusieurs signes cliniques et biologiques qui accroissent le risque de maladies cardiaques, d'accidents cardiovasculaires et de diabète de type 2.

Dans le cas d'une obésité morbide et sévère, il peut être nécessaire d'avoir recours à la chirurgie bariatrique qui permet de restreindre l'absorption des aliments, notamment en réduisant la capacité gastrique, diminuant ainsi l'apport calorique journalier, ou en provoquant une malabsorption.

Il existe plusieurs types d'interventions de chirurgie bariatrique, telle que la pose d'anneau, sleeve, dérivation biliopancréatique ou by-pass. Toutes ces techniques, qui sont pratiquées par incision de la paroi abdominale pour une chirurgie laparoscopique, sont difficiles à mettre en oeuvre, car la présence de la masse grasse viscérale autour du foie et la stéatose du foie gênent le chirurgien lors de son intervention en limitant l'accès à l'estomac.

C'est pourquoi, il est essentiel de pouvoir, en préopératoire bariatrique, faire diminuer la graisse viscérale et la taille du foie stéatosé car on sait que les patients en obésité sévère ont une stéatohépatite non alcoolique ou NASH.

La solution actuelle consiste à imposer une diète stricte au patient qui va subir l'intervention. Toutefois, cette diète est difficile à respecter pour le patient qui va subir l'intervention, et entraîne en outre des risques de sténose hépatique. De plus, il est démontré que ce type de régime augmente les compulsions alimentaires et la tendance à la dépression en augmentant la déficience en tryptophane déjà existante chez l'obèse, provoquée par l'inflammation chronique et le stress. Enfin, cette diète aggrave les déficiences symptomatiques des personnes en surpoids.

Par ailleurs, les personnes obèses souffrent de nombreuses déficiences en micronutriments, en particulier en micronutriments anti-inflammatoires comme le zinc, le chrome, les omégas 3, l'arginine et la taurine qui gênent la cicatrisation. En outre, avec une obésité abdominale les patients ont un taux élevé de fibrogène favorisant la survenance de thrombose post opératoire.

Enfin, le patient arrive en salle opératoire avec un stress important qui s'ajoute à son stress naturel.

Tous ces phénomènes liés à l'état d'obésité du patient, vont accentuer les difficultés d'intervention bariatrique s'ils ne sont pas traités, et une diète stricte, à moins de 800 kcal/jour, avant l'opération ne fera que les aggraver.

De plus, il est essentiel que la perte de poids avant l'opération ne se traduise pas par une perte de masse maigre car après l'intervention la perte de plusieurs kilogrammes par semaine se traduira par une perte importante de masse maigre qui affecte le métabolisme de base et induit souvent une reprise de poids. De plus, la diminution de masse maigre affecte aussi un organe comme le coeur ce qui peut être dangereux lors de l'anesthésie et l'intervention en général.

WO2010/114627 A1 décrit une boisson à base de lait comprenant du caséinate de sodium et un isolat de lactosérum et qui est destinée aux intolérants au lactose.

Il subsiste donc un besoin pour une solution efficace, naturelle et facile d'utilisation, qui soit capable de diminuer spécifiquement le gras viscéral et répondre aux déficiences et carences des personnes obèses en phase préopératoire d'une intervention chirurgicale bariatrique.

Pour y répondre, la présente invention propose d'utiliser une composition particulière comprenant un mélange de principes actifs constitué par au moins :
- un hydrolysat de lactosérum de poids moléculaire compris entre 200 et 10 000 daltons,
- un isolat et/ou un concentrat de lactosérum, et
- du caséinate de calcium.

Avantageusement, un telle composition peut être utilisée comme produit diététique destiné notamment à faire diminuer le gras viscéral chez des personnes obèses en phase préopératoire d'une intervention chirurgicale bariatrique, et permet de faire perdre entre 25 et 50% en volume du foie.

Par ailleurs une telle composition permet de lutter contre des déficiences et carences spécifiques aux personnes obèses, et ainsi diminuer les risques d'une opération bariatrique.

L'invention est à présent décrite en détails.

L'invention vise donc une composition diététique pour son utilisation comme produit diététique ou pour la préparation d'un produit diététique à administration orale pour la diminution du gras viscéral chez une personne obèse en phase préopératoire d'une intervention de chirurgie bariatrique, comprenant un mélange de principes actifs constitué par au moins :
- un hydrolysat de lactosérum de poids moléculaire compris entre 200 et 10 000 daltons,
- un isolat et/ou un concentrat de lactosérum, et
- du caséinate de calcium.

Par composition ou produit diététique au sens de l'invention, on entend un produit destiné à une alimentation particulière, en complément d'un régime restrictif ou d'une alimentation équilibrée. La composition ou le produit diététique selon l'invention est particulièrement adapté aux personnes qui souhaitent se préparer efficacement à une opération bariatrique en limitant les risques opératoires et en ayant une meilleure récupération.

Par hydrolysat de lactosérum au sens de l'invention on entend toute molécule ou mélange de molécules obtenu(e) par un procédé comprenant un étape d'hydrolyse chimique ou d'hydrolyse enzymatique de lactosérum.

Par isolat de lactosérum au sens de l'invention on entend un extrait de lactosérum qui contient moins de 1% de lactose et de matières grasses.

Par concentrat de lactosérum au sens de l'invention on entend un extrait de lactosérum obtenu par concentration d'un lactosérum.

L'hydrolysat de lactosérum a un poids moléculaire compris entre 200 et 10 000 Daltons, préférentiellement entre 200 et 3500 daltons. Il est essentiellement composé de di et tri peptides.

De façon préférée, il s'agit d'un hydrolysat peptidique de lactosérum comprenant au moins 90% de peptides en poids de matière sèche de l'hydrolysat.

L'isolat et/ou le concentrat de lactosérum ont préférentiellement un poids moléculaire compris entre 15 000 et 20 000 daltons.

L'isolat de lactosérum est préférentiellement fabriqué à partir de lait frais et de fromageries qui ne pasteurisent pas le lait pour éviter la destruction des bêta-lactoglobuline et alpha-lactalbumine, et qui extraient le lactosérum par ultrafiltration ou microfiltration (taille des filtres de 0,1µm). L'isolat obtenu par échange d'ions est moins adapté en raison de sa faible teneur en bêta-lactoglobuline et alpha-lactalbumine. L'isolat contient moins de 1% de lactose et de matières grasses, et sa concentration en peptides est préférentiellement d'au moins 90% en poids de matière sèche.

Le concentrat de lactosérum est préférentiellement obtenu à partir d'un lactosérum de fromagerie sans pasteurisation, contenant des bêta-lactoglobuline, des alpha-lactalbumine et des glycomacropeptides. La concentration en peptides du concentrat est préférentiellement d'au moins 80% en poids de matière sèche.

Par ailleurs, le caséinate de calcium utilisé dans la composition selon l'invention a préférentiellement un poids moléculaire compris entre 20 000 et 35 000 daltons.

Avantageusement, les différents constituants de la composition agissent en synergie. La présence de l'hydrolysat particulier associée à celle de l'isolat et/ou du concentrat de lactosérum permet notamment d'accélérer la perte du gras viscéral et joue sur la sensation de satiété. Le caséinate de calcium quant à lui a notamment un effet coupe-faim.

De façon préférée, le ratio en poids entre le caséinate de calcium et le mélange constitué par l'hydrolysat et l'isolat et/ou le concentrat de lactosérum est de 0,8 à 1,2 dans la composition. Une telle caractéristique favorise encore la perte de gras viscéral.

Selon un mode de réalisation particulièrement adapté, le mélange de principes actifs de la composition comprend également un mélange d'acides aminés. La présence d'acides aminés permet d'améliorer l'efficacité de la composition diététique selon l'invention.

Les acides aminés présents dans la composition sont préférentiellement au moins le tryptophane, la glutamine, la leucine, l'arginine et/ou la taurine, mais la composition peut contenir d'autres acides aminés, comme l'isoleucine, la valine, la phénylalanine ou la tyrosine. Très préférentiellement, la composition selon l'invention comprend au moins le tryptophane, la leucine, l'arginine et la taurine. Lorsque le tryptophane est présent, il doit représenter entre 6 et 9%, préférentiellement environ 7% en poids des acides aminés neutres présents dans la composition (leucine, isoleucine, valine, phénylalanine, tyrosine et tryptophane). Cette proportion particulière permet d'assurer qu'une quantité adaptée de tryptophane puisse traverser la barrière hématoencéphalique pour être transformée en sérotonine de façon notamment à agir sur la sensation de satiété en complément des actions de l'hydrolysat de lactosérum, de l'arginine et de la taurine couplées au zinc pour les hormones incrétines et à favoriser la gestion du stress.

Lorsque l'arginine et la taurine sont présentes, le rapport du poids d'arginine sur le poids de taurine est compris entre 1,5 et 2.

En plus du mélange hydrolysat, isolat et/ou concentrat de lactosérum et caséinate de calcium, et des acides aminés, le mélange de principes actifs de la composition selon l'invention peut comprendre un ou plusieurs des éléments choisis parmi le calcium de lait, le magnésium, la vitamine B6, la vitamine B9, la vitamine E, la vitamine D, le zinc et le chrome.

De même, la composition peut contenir des acides gras essentiels, notamment des omégas 3. Préférentiellement, il s'agit d'omégas 3 d'origine végétale, avec une proportion élevée d'EPA.

Les différents constituants de la composition selon l'invention agissent en synergie pour procurer des effets surprenants particulièrement adaptés à la diminution du gras viscéral en phase préopératoire d'une intervention de chirurgie bariatrique.

Selon un mode de réalisation préféré, le mélange de principes actifs de la composition selon l'invention comprend au moins :
- 8 à 12 % d'hydrolysat de lactosérum,
- 15 à 20 % d'isolat et/ou de concentrat de lactosérum,
- 20 à 25 % de caséinate de calcium.
les pourcentages étant donnés en poids de matière sèche de la totalité des principes actifs présents de la composition (en dehors des éventuels excipients). La composition peut également contenir des éléments ajoutés librement, tels que des acides aminés, des vitamines et des minéraux, qui viennent s'ajouter aux constituants natifs de l'hydrolysat de lactosérum, de l'isolat de lactosérum, du concentrat de lactosérum et du caséinate de calcium.

La composition selon l'invention est préférentiellement constituée par au moins :
- 1,5 à 3% de tryptophane,
- 12 à 20 % d'acides aminés branchés,
- 6 à 10 % d'acides aminés aromatiques,
- 0,8 à 1,5% de taurine,
- 1,6 à 3% d'arginine,
- 1,2 à 3% de calcium de lait,
- 0,5 à 1% de magnésium,
- 0,4 à 1% d'omégas 3,
- 1 à 2 mg de vitamine B6 pour 50g de composition sans excipients,
- 5 à 15mg de zinc pour 50g de composition sans excipients,
- 1 à 3µg de vitamine D pour 50g de composition sans excipients,
- 75 à 150 µg de chrome pour 50g de composition sans excipients,
- 100 µg de vitamine B9 pour 50g de composition sans excipients,
- 10mg de vitamine E pour 50g de composition sans excipients.
les pourcentages étant donnés en poids de matière sèche de la totalité des principes actifs présents de la composition (en dehors des éventuels excipients), une partie des constituants provenant de l'hydrolysat de lactosérum, de l'isolat de lactosérum, du concentrat de lactosérum et du caséinate de calcium et le reste étant ajouté librement sous forme d'acides aminés, vitamines et minéraux. Les acides aminés branchés de la composition sont constitués de leucine, d'isoleucine et de valine, préférentiellement de :
- 50 à 60% de leucine,
- de 18 à 25% d'isoleucine, et
- de 20 à 28% de valine,
et les acides aminés aromatiques de tryptophane, de phénylalanine et de tyrosine, préférentiellement de :
- 15 à 24% de tryptophane,
- de 38 à 46% de phénylalanine, et
- de 35 à 43% de tyrosine.

La composition selon l'invention peut être obtenue par un procédé tel que décrit en suivant :
- un premier mélange est obtenu par mélange des constituants dans l'ordre suivant : caséinate de calcium, isolat de lactosérum, concentrat de lactosérum, hydrolysat de lactosérum, acides aminés libres, magnésium et calcium de lait. Le pH doit être aux alentours de 7 et stabilisé à ce niveau.
- ajout au premier mélange des vitamines, des minéraux et des acides gras.

On obtient ainsi une poudre qui peut être transformée en comprimé ou liquide, ou bien utilisée dans sa forme poudre en sachets, sticks, bidons ou gélules par exemple.

La composition selon l'invention, lorsqu'elle est administrée par voie orale en quantité suffisante, permet d'agir directement sur la perte du gras, en particulier grâce à la présence du mélange d'hydrolysat et d'isolat et/ou de concentrat de lactosérum.

Elle agit aussi comme coupe-faim et procure une sensation de satiété, notamment par l'action de l'hydrolysat de lactosérum. Cet effet est amélioré en présence de tryptophane, et peut être encore accentué grâce à la présence d'autres constituants, en particulier de calcium de lait, d'histidine, de vitamine B6 et/ou de magnésium, les différents constituants agissant alors en synergie.

La composition selon l'invention est particulièrement adaptée pour diminuer la graisse viscérale autour du foie chez un patient avant une opération de chirurgie bariatrique et pour diminuer la taille du foie lui aussi chargé en graisse. Une cure particulièrement adaptée pour la préparation à une intervention chirurgicale de pose d'anneau, de sleeve, de by-pass gastrique ou de dérivation biliopancréatique, est de 6 semaines avec une restriction alimentaire qui soit de 20 à 40% en dessous de la DER (Dépense Energétique de Repos) encore appelé métabolisme de base de la personne qui suit la cure. La diminution du gras viscéral ainsi obtenue, permet au chirurgien de pratiquer au mieux son opération et de diminuer de 5 à 10% le temps de l'intervention.

La composition selon l'invention permet également de perdre durablement du poids en particulier par l'apport de tryptophane qui régule la satiété en synergie avec l'action sur l'inflammation de la taurine, arginine, zinc, chrome par une régulation du signal NF Kappa B des macrophages des adipocytokines et de régulation des hormones incrétines. De plus, elle diminue le tour de taille, tout en maintenant la masse maigre notamment par l'apport d'acides aminés branchés.

Selon un autre avantage, la composition est également capable d'agir sur d'autres facteurs du risque opératoire. En particulier elle est capable de :
- réduire le stress et la dépression ainsi que de normaliser la tension artérielle qui sont des facteurs aggravant des risques de l'anesthésie,
- diminuer l'inflammation CRP ultrasensible qui permet d'améliorer le temps de cicatrisation,
- réguler le facteur de coagulation fibrinogène qui permet d'éviter les thromboses,
- commencer à traiter une éventuelle stéatose hépatique par la perte de gras viscéral associée à la vitamine E,
- lutter contre la déficience en tryptophane avant l'opération, le manque de tryptophane chez les patients obèses dépressifs ralentissant sérieusement la perte de poids et facteur essentiel de la reprise de poids après l'opération.

La composition selon l'invention peut se présenter sous toute forme adaptée à une administration par voie orale. Elle peut notamment se présenter sous forme de poudre ou granulés, de boissons prêtes à l'emploi, de barres ou d'extrudés, la composition étant additionnée d'excipients et charges classiques connues de l'homme du métier.

Préférentiellement, elle se présente sous forme de poudre ou granules conditionnée dans un sachet à diluer dans l'eau.

La dose journalière de composition selon l'invention (dose de mélange de principes actifs sans les excipients) est préférentiellement comprise entre 66 et 110 g, de préférence en deux prises de 33 à 55 g réparties une le matin au petit déjeuner ou à 11h en collation, et une en collation l'après-midi. Avantageusement, la biodisponibilité dans l'organisme des acides aminés, peptides et protéines présents dans la composition dans l'organisme est comprise entre 10 minutes et 5 heures, ce qui permet une action à la fois rapide et qui perdure dans le temps de façon à limiter la quantité de nourriture à prendre quotidiennement.

En outre, la présence de calcium de lait permet d'améliorer la palabilité du produit diététique selon l'invention en masquant notamment le goût amer de l'hydrolysat de lactosérum, de sorte qu'il participe à supprimer le risque que les personnes cessent de le consommer pour des raisons de goût et abandonnent leur régime avant son terme.

La composition diététique permet de diminuer le gras viscéral chez les personnes en surcharge pondérale, notamment par l'accélération du processus de lipolyse, la régulation de la triade oxydation-inflammation-coagulation et l'apport d'actifs palliant les déficiences pathologiques des personnes obèses.

La combinaison des acides gras, en particulier des omégas 3, avec le calcium de lait et le mélange d'acides aminés particulier selon l'invention, permet également de ralentir la transformation des pré-adipocytes en adipocytes viscéraux.

L'invention est à présent illustrée par un exemple non limitatif de composition diététique, se présentant sous forme d'une poudre de 55 g (principes actifs et excipients) conditionnée dans un sachet.

Cette composition est obtenue à partir des principes actifs suivants :
- 5g d'hydrolysat de lactosérum de poids moléculaire entre 200 et 3500 daltons,
- 10 g d'isolat et/ou de concentrat de lactosérum de poids moléculaire entre 15000 et 20000 daltons,
- 13 g de caséinate de calcium de poids moléculaire entre 20000 et 35000 daltons,
- 1 mg de vitamine B6,
- 10 mg de zinc,
- 0,45 g de taurine,
- 40 µg de chrome,
- 10 mg de vitamine E,
- 100 µg de vitamine B9,
- 2,5 µg de vitamine D,
- 270 mg d'omégas 3 d'origine végétale,
- qsp 4,2g de leucine,
- qsp 0,8g de tryptophane,
- qsp 0,9g d'arginine,
- qsp 0,75g de calcium de lait,
- qsp 0,36 g de magnésium.

Par « qsp *Xg* » d'un élément de la composition, on entend la quantité totale de cet élément dans la composition : quantité apportée par les protides (caséinate de calcium, isolat de lactosérum, concentrat de lactosérum, hydrolysat de lactosérum) et complétée par un ajout de l'élément sous forme libre pour arriver jusqu'à *Xg.*

L'invention est à présent illustrée par une étude montrant l'effet de la composition sur la diminution du gras viscéral sur d'autres facteurs du risque opératoire bariatrique.

L'étude a été réalisée sur des patients :
- présentant un tour de taille excessif par rapport aux normes IDF 2006 (80cm pour les femmes et 94 cm pour les hommes),
- présentant au moins deux facteurs à risque cardiométabolique choisi parmi : pression artérielle élevée, glycémie élevée, dyslipidémie (triglycérides élevés, cholestérol total et LDL élevés, HDL bas), tabagismes et antécédents familiaux.

Les patients ont suivi un régime adapté à leurs habitudes alimentaires, équilibré (50% de glucides, 35% de lipides, 15% de protéines ; glucides à charge glycémique inférieure à 10), hypocalorique (restriction de 700 kcal sur la Dépense Energétique Totale [DET] calculée) et comportant deux prises par jour d'une composition selon l'invention (celle de l'exemple) apportant 360 kcal pris en compte dans la ration journalière. Une activité physique d'un minimum de 5000 pas (mesurée au podomètre) était prescrite. Cette phase intensive devait être arrêtée lorsqu'était atteinte une diminution de 10% du tour de taille, ou lorsque celui-ci revenait dans les normes IDF. Elle devait être interrompue au bout de 9 mois si l'objectif n'était pas atteint.

Cette phase intensive devait être suivie d'une phase de stabilisation comportant une alimentation équilibrée sans restriction calorique avec une dose de composition selon l'invention (celle de l'exemple) par jour. 92 patients ont été inclus dans l'étude au départ : 28 ont été perdus de vue après l'inclusion, 64 ont terminé la phase d'intervention nutritionnelle intensive et 34 ont participé à la phase de stabilisation.

Les données de base des 64 patients inclus et ayant terminé la phase nutritionnelle intensive de l'étude sont présentés dans le tableau suivant :

**Tableau 1**

| | Patients ayant terminé la phase d'intervention nutritionnelle (moyenne ± écart type ou nombre de patients) N=64 | |
|---|---|---|
| | Hommes | Femmes |
| | 15 | 49 |
| Age moyen (années) | 53.7 ±11.1 | 53.0 ±11.0 |
| Tour de taille (cm) | 101.9 ±7.3 | 91.9 ±10.2 |
| Taille (cm) | 177.8 ±4.9 | 163.6 ±6.1 |
| Poids initial (kg) | 92.3 ±8.6 | 74.3 ±11.5 |
| IMC (kg /m²) | 29.2 ±2.6 | 27.8 ±4.9 |
| Inclusion tour de taille hors normes avec | | |
| IMC<25 | 0 | 13 |
| avec IMC>25 | 36 | 15 |
| dont obèses (*(IMC> 30)* | 4 | 11 |
| DER métabolisme de base (Kcal) | | |
| | 1745 ± 272 | 1367 ±110 |
| Masse grasse (kg) | 27.6 ±7.4 | |
| Masse maigre (kg) | 50.9 ±10.0 | |

Les résultats obtenus pour les 64 patients ayant terminé la phase nutritionnelle intensive sont présentés dans le tableau suivant :

**Tableau 2**

| | Valeur initiale (moyenne ± écart type) | Valeur finale | Evolution (moyenne ± écart type) | % évolution |
|---|---|---|---|---|
| DER (Kcal) | 1455 ±227 | 1439.5 ±201.5 | -15.8 ±117 | -0.6 |
| Tour de taille (cm) | 94.2 ±10.5 | 85.0 ±7.5 | -9.2 ±6.8 | -9.4 |
| Poids (kg) | 78.5 ±13.3 | 69.3 ±10.6 | -9.2 ±5.3 | -11.4 |
| IMC | 28.2 ±4.5 | 24.8 ±3.0 | -3.3 ±2.1 | -11.4 |
| Masse grasse (kg) | 27.6 ±7.4 | 20.0 ±4.9 | -7.5 ±6.2 | -25.8 |
| Masse maigre (kg) | 50.9 ±10.0 | 49.3 ±9.9 | -1.6 ±3.2 | -3.1 |
| Tension artérielle systolique (mm Hg) | 128 ±15.8 | 117.6 ±12.1 | -10.5 ±14.1 | -7.4 |
| diastolique (mmHg) | 80.2 ±10.8 | 70.0 ±6.1 | -10.3 ±9.7 | -11.7 |
| Cholestérol total (mmol/l) | 5.7 ±1.0 | 5.1 ±0.7 | -0.6 ±0.6 | -9.4 |
| HDL (mmol/l) | 1.5 ±0.5 | 1.5 ±0.4 | 0 ±0.1 | 0.7 |
| LDL (mmol/l) | 3.6 ±0.9 | 3.3 ±0.8 | -0.3 ±0.5 | -7.9 |
| CT/HDL | 4.2 ±2.4 | 3.6 ±1.8 | -0.5 ±0.8 | -9.4 |
| Triglycerides (mmol/l) | 1.2 ±1.0 | 1.0 ±0.3 | -0.2 ±0.8 | -7.5 |
| Glycémie (mmol/l) | 5.8 ±2.8 | 5.1 ±0.4 | -0.7 ±2.7 | -4.8 |
| Insulinémie (µU/ml) | 10.5 ±6.9 | 7.0 ±4.6 | -3.5 ±3.1 | -30.1 |
| HOMA-IR | 3.2 ±3.8 | 1.6 ±1.1 | -1.6 ±3.2 | -35 |
| CRP us (mg/l) | 2.3 ±3.1 | 1.4 ±1.6 | -0.9 ±2.1 | -17.8 |
| Fibrinogène (g/l) | 3.6 ±0.8 | 3.2 ±0.6 | -0.5 ±0.5 | -11.5 |
| Créatinine (mg/l) | 7.8 ±1.8 | 7.6 ±1.2 | -0.2 ±1.2 | / |
| Urée (g/l) | 0.4 ±0.1 | 0.4 ±0.4 | 0 ±0.4 | / |

Ces résultats montrent une perte pondérale de plus de 11%. Le nombre de patients avec un IMC < 25 est passé de 13 à l'inclusion à 41 à la fin de l'étude. La perte de masse grasse par rapport à la masse maigre a été prépondérante, portant le rapport de la masse maigre sur le poids total de 65% à 71%.

On constate également une perte moyenne de presque 10% du tour de taille.

De plus, la composition selon l'invention permet une diminution importante des autres facteurs du risque opératoire bariatrique, notamment :
- pression artérielle : la pression artérielle est améliorée. Elle a été normalisée chez la totalité des 17 hypertendus inclus.
- paramètres biologiques : les paramètres biologiques ont été améliorés, en particulier :
   - le profil lipidique : 11 patients sur 19 ont normalisé leur LDL à l'issue de la phase d'amaigrissement
   - les marqueurs de l'inflammation : 10 patients qui avaient une CPR us élevée l'ont normalisée ainsi que 4 patients pour le fibrinogène ce qui est inhabituel
   - les marqueurs de l'insulinorésistance : 10 patients pour la glycémie et 4 patients pour l'insulinémie qui étaient hors normes ont régulé le risque de diabète ; de plus pour l'ensemble des sujets il a été constaté une baisse de 35% de l'insulinorésistance caractérisée par le HOMA-IR3
   - la créatinine a été en baisse et l'urée est restée stable, ce qui suggère que le protocole nutritionnel utilisé dans l'étude n'a pas d'impact détectable sur la fonction rénale.

La baisse de 10% du tour de taille entraine une baisse de la taille du foie par perte de masse grasse hépatocytaire. Ceci permet une meilleure accessibilité de l'estomac pour le chirurgien mais surtout prépare parfaitement le patient en éliminant les carences qui pourraient provoquer des problèmes post opératoires comme mauvaise cicatrisation ou oedème. De plus la perte de gras viscéral avant l'opération entraine une plus grande perte à un an de l'intervention.

Par ailleurs, avantageusement, en 6 mois aucun effet indésirable n'a été rapporté spontanément.

## Revendications

1. Composition diététique, pour son utilisation comme produit diététique à administration orale pour la diminution du gras viscéral chez une personne obèse en phase préopératoire d'une intervention de chirurgie bariatrique, comprenant un mélange de principes actifs constitué par au moins :
- un hydrolysat de lactosérum de poids moléculaire compris entre 200 et 10 000 daltons,
- un isolat et/ou un concentrat de lactosérum, et
- du caséinate de calcium.

2. Composition selon la revendication 1, **caractérisée en ce que** l'isolat et/ou le concentrat ont un poids moléculaire compris entre 15 000 et 20 000 daltons.

3. Composition selon l'une des précédentes revendications, **caractérisée en ce que** le caséinate de calcium a un poids moléculaire compris entre 20 000 et 35 000 daltons.

4. Composition selon l'une des précédentes revendications, **caractérisée en ce que** l'hydrolysat de lactosérum a un poids moléculaire compris entre 200 et 3500 daltons.

5. Composition selon l'une des précédentes revendications, **caractérisée en ce que** le ratio en poids entre le caséinate de calcium et le mélange constitué par l'hydrolysat et l'isolat et/ou le concentrat de lactosérum est compris entre 0,8 et 1,2.

6. Composition selon l'une des précédentes revendications, **caractérisée en ce que** le mélange de principes actifs comprend également un mélange d'acides aminés.

7. Composition selon la revendication 6, caractérisée en que le mélange d'acides aminés est constitué par au moins un des acides aminés choisis parmi le tryptophane, la glutamine, la leucine, l'arginine et la taurine.

8. Composition selon la revendication 7, caractérisée en que le tryptophane représente entre 6 et 9% en poids des acides aminés neutres présents dans la composition.

9. Composition selon la revendication 7 ou 8, **caractérisée en ce que** le rapport du poids d'arginine sur le poids de taurine est compris entre 1,5 et 2.

10. Composition selon l'une des précédentes revendications, **caractérisée en ce que** le mélange de principes actifs comprend également au moins un des éléments choisis parmi le calcium de lait, le magnésium, la vitamine B6, la vitamine B9, la vitamine E, la vitamine D, le zinc et le chrome.

11. Composition selon l'une des précédentes revendications, **caractérisée en ce que** le mélange de principes actifs comprend également des acides gras essentiels.

12. Composition selon la revendication 11, **caractérisée en ce que** les acides gras essentiels sont des omégas 3.

13. Composition selon l'une des précédentes revendications, **caractérisée en ce que** l'hydrolysat de lactosérum représente entre 8 et 12%, l'isolat et/ou le concentrat de lactosérum entre 15 et 20%, et le caséinate de calcium entre 20 et 25%, les pourcentages étant donnés en poids de matière sèche de la totalité des principes actifs présents de la composition.

14. Composition selon l'une des précédentes revendications, **caractérisée en ce qu'**elle est constituée par au moins :
- 1,5 à 3% de tryptophane,
- 12 à 20 % d'acides aminés branchés,
- 6 à 10 % d'acides aminés aromatiques,
- 0,8 à 1,5% de taurine,
- 1,6 à 3% d'arginine,
- 1,2 à 3% de calcium de lait,
- 0,5 à 1% de magnésium,
- 0,4 à 1% d'omégas 3,
- 1 à 2 mg de vitamine B6 pour 50g de composition sans excipients,
- 5 à 15 mg de zinc pour 50g de composition sans excipients,
- 1 à 3 µg de vitamine D pour 50g de composition sans excipients,
- 75 à 150 µg de chrome pour 50g de composition sans excipients,
- 100 µg de vitamine B9 pour 50g de composition sans excipients,
- 10 mg de vitamine E pour 50g de composition sans excipients,
les pourcentages étant donnés en poids de matière sèche de la totalité des principes actifs présents de la composition.

15. Composition selon l'une des précédentes revendications, **caractérisée en ce qu'**elle se présente sous forme de poudre ou granulés, de boisson prête à l'emploi, de barres alimentaires ou extrudés.

## Patentansprüche

1. Diätetische Zusammensetzung für deren Verwendung als diätetisches Produkt zur oralen Anwendung für die Verringerung von viszeralem Fettgewebe bei einer adipösen Person in der präoperativen Phase einer Intervention der bariatrischen Chirurgie, die eine Mischung von Wirkstoffen aufweist, die sich wenigstens zusammensetzt aus:
- einem Hydrolysat aus Milchserum mit einem Molekulargewicht zwischen 200 und 10.000 Daltons,
- einem Isolat und/oder einem Konzentrat aus Milchserum, und
- einem Kalziumkaseinat.

2. Zusammensetzung nach Anspruch 1, **dadurch gekennzeichnet, dass** das Isolat und/oder das Konzentrat ein Molekulargewicht zwischen 15.000 und 20.000 Daltons hat.

3. Zusammensetzung nach einem der vorhergehenden Ansprüche, **dadurch gekennzeichnet, dass** das Kalziumkaseinat ein Molekulargewicht zwischen 20.000 und 35.000 Daltons hat.

4. Zusammensetzung nach einem der vorhergehenden Ansprüche, **dadurch gekennzeichnet, dass** das Hydrolysat aus Milchserum ein Molekulargewicht zwischen 200 und 3.500 Daltons hat.

5. Zusammensetzung nach einem der vorhergehenden Ansprüche, **dadurch gekennzeichnet, dass** das Gewichtsverhältnis zwischen dem Kalziumkaseinat und der aus dem Hydrolysat und dem Isolat und/oder dem Konzentrat aus Milchserum bestehenden Mischung zwischen 0,8 und 1,2 liegt.

6. Zusammensetzung nach einem der vorhergehenden Ansprüche, **dadurch gekennzeichnet, dass** die Mischung der Wirkstoffe auch eine Mischung von Aminosäuren aufweist.

7. Zusammensetzung nach Anspruch 6, **dadurch gekennzeichnet, dass** die Mischung aus Aminosäuren aus wenigstens einer der Aminosäuren zusammengesetzt ist, die unter Tryptophan, Glutamin, Leucin, Arginin und Taurin ausgewählt sind.

8. Zusammensetzung nach Anspruch 7, **dadurch gekennzeichnet, dass** Tryptophan zwischen 6 und 9 Gew.-% der in der Zusammensetzung vorhandenen neutralen Aminosäuren darstellt.

9. Zusammensetzung nach Anspruch 7 oder 8, **dadurch gekennzeichnet, dass** das Verhältnis des Gewichts von Arginin zum Gewicht von Taurin zwischen 1,5 und 2 liegt.

10. Zusammensetzung nach einem der vorhergehenden Ansprüche, **dadurch gekennzeichnet, dass** die Mischung aus Wirkstoffen auch wenigstens einen der Bestandteile aufweist, die aus Milchkalzium, Magnesium, Vitamin B6, Vitamin B9, Vitamin E, Vitamin D, Zink und Chrom ausgewählt sind.

11. Zusammensetzung nach einem der vorhergehenden Ansprüche, **dadurch gekennzeichnet, dass** die Mischung der Wirkstoffe auch essentielle Fettsäuren aufweist.

12. Zusammensetzung nach Anspruch 11, **dadurch gekennzeichnet, dass** die essentiellen Fettsäuren Omega-3-Fettsäuren sind.

13. Zusammensetzung nach einem der vorhergehenden Ansprüche, **dadurch gekennzeichnet, dass** das Hydrolysat aus Milchserum zwischen 8 und 12%, das Isolat und/oder das Konzentrat aus Milchserum zwischen 15 und 20% und das Kalziumkaseinat zwischen 20 und 25% darstellt, wobei die Prozentangaben auf die Trockenmasse der gesamten, in der Zusammensetzung vorhandenen Wirkstoffe bezogen sind.

14. Zusammensetzung nach einem der vorhergehenden Ansprüche, **dadurch gekennzeichnet, dass** diese sich wenigstens zusammensetzt aus:
- 1,5 bis 3% Tryptophan,
- 12 bis 20% verzweigte Aminosäuren,
- 6 bis 10% aromatische Aminosäuren,
- 0,8 bis 1,5% Taurin,
- 1,6 bis 3% Arginin,
- 1,2 bis 3% Milchkalzium,
- 0,5 bis 1% Magnesium,
- 0,4 bis 1% Omega-3-Fettsäuren,
- 1 bis 2 mg Vitamin B6 je 50 g Zusammensetzung ohne Hilfsstoffe,
- 5 bis 15 mg Zink je 50 g Zusammensetzung ohne Hilfsstoffe,
- 1 bis 3 µg Vitamin D je 50 g Zusammensetzung ohne Hilfsstoffe,
- 75 bis 150 µg Chrom je 50 g Zusammensetzung ohne Hilfsstoffe,
- 100 µg Vitamin B9 je 50 g Zusammensetzung ohne Hilfsstoffe,
- 10 mg Vitamin E je 50 g Zusammensetzung ohne Hilfsstoffe,
wobei die Prozentangaben auf die Trockenmasse der gesamten, in der Zusammensetzung vorhandenen Wirkstoffe bezogen sind.

15. Zusammensetzung nach einem der vorhergehenden Ansprüche, **dadurch gekennzeichnet, dass** diese als Pulver oder Granulat, als Fertiggetränk, als Nahrungsriegel oder stranggepresster Riegel ausgebildet ist.

## Claims

1. Dietary composition, for its use as an orally-administered dietary product for the reduction of visceral fat in an obese individual during the pre-operative phase of a bariatric surgery intervention, comprising a mixture of active ingredients consisting of at least:
- A whey hydrolyzate with a molecular weight of between 200 and 10,000 daltons,
- An isolate and/or a concentrate of whey, and
- Calcium caseinate.

2. Composition according to Claim 1, **characterized in that** the isolate and/or the concentrate has/have a molecular weight of between 15,000 and 20,000 daltons.

3. Composition according to one of the preceding claims, wherein the calcium caseinate has a molecular weight of between 20,000 and 35,000 daltons.

4. Composition according to one of the preceding claims, wherein the whey hydrolyzate has a molecular weight of between 200 and 3,500 daltons.

5. Composition according to one of the preceding claims, wherein the ratio by weight between calcium caseinate and the mixture consisting of the hydrolyzate and the isolate and/or the concentrate of whey is between 0.8 and 1.2.

6. Composition according to one of the preceding claims, wherein the mixture of active ingredients also comprises a mixture of amino acids.

7. Composition according to Claim 6, wherein the mixture of amino acids consists of at least one of the amino acids selected from among tryptophan, glutamine, leucine, arginine, and taurine.

8. Composition according to Claim 7, wherein tryptophan represents between 6 and 9% by weight of the neutral amino acids that are present in the composition.

9. Composition according to Claim 7 or 8, wherein the ratio of the weight of arginine to the weight of taurine is between 1.5 and 2.

10. Composition according to one of the preceding claims, wherein the mixture of active ingredients also comprises at least one of the elements selected from among milk calcium, magnesium, vitamin B6, vitamin B9, vitamin E, vitamin D, zinc, and chromium.

11. Composition according to one of the preceding claims, wherein the mixture of active ingredients also comprises essential fatty acids.

12. Composition according to Claim 11, wherein the essential fatty acids are omega-3 fatty acids.

13. Composition according to one of the preceding claims, wherein the whey hydrolyzate represents between 8 and 12%, the isolate and/or the concentrate of whey between 15 and 20%, and calcium caseinate between 20 and 25%, with the percentages being given by weight of dry material of all of the active ingredients that are present in the composition.

14. Composition according to one of the preceding claims, wherein it consists of at least:
- 1.5 to 3% tryptophan,
- 12 to 20% branched amino acids,
- 6 to 10% aromatic amino acids,
- 0.8 to 1.5% taurine,
- 1.6 to 3% arginine,
- 1.2 to 3% milk calcium,
- 0.5 to 1% magnesium,
- 0.4 to 1% omega-3 fatty acids,
- 1 to 2 mg of vitamin B6 per 50 g of the composition without vehicles,
- 5 to 15 mg of zinc per 50 g of composition without vehicles,
- 1 to 3 µg of vitamin D per 50 g of composition without vehicles,
- 75 to 150 µg of chromium per 50 g of composition without vehicles,
- 100 µg of vitamin B9 per 50 g of composition without vehicles,
- 10 mg of vitamin E per 50 g of composition without vehicles,
with the percentages being given by weight of dry material of all of the active ingredients that are present in the composition.

15. Composition according to one of the preceding claims, wherein it comes in the form of powder or granules, ready-to-use drink, food bars or logs.
